# EUROPEAN PATENT SPECIFICATION

(11) **EP 3 214 077 B1**
(45) Date of publication and mention of the grant of the patent: **11.03.2020**
(21) Application number: 16460012.4
(22) Date of filing: 01.03.2016
(51) Int. Cl.: C07D 295/135, A61K 31/445, A61P 3/10

(54) **PROCESS FOR THE PURIFICATION OF A PHARMACEUTICAL AGENT**
VERFAHREN ZUR REINIGUNG EINES PHARMAZEUTISCHEN WIRKSTOFFS
PROCÉDÉ DE PURIFICATION D'UN AGENT PHARMACEUTIQUE

(43) Date of publication of application: 06.09.2017
(73) Proprietor: Zaklady Farmaceutyczne Polpharma S.A., 83-200 Starogard Gdanski (PL)
(72) Inventor: Szramka, Roman, 83-140 Gniew (PL); Ignatowicz, Dawid, 80-293 Gdansk (PL)
(74) Representative: Obrycki, Pawel Andrzej

(56) References cited:
- WO-A2-2004/101540
- WO-A2-2009/004485

## Description

The present invention relates to a process for the purification of a pharmaceutical agent, and particularly to a process for purifying repaglinide.

Repaglinide is an anti-diabetic used for the treatment of diabetes mellitus type II (also referred to as non-insulin-dependent diabetes mellitus (NIDDM) or adult-onset diabetes). Type II diabetes is a milder, heterogeneous form of diabetes and often presents more frequently in older age. The disease results from relative insulin deficiency and insulin resistance. These problems frequently arise from, and are exacerbated by, obesity which causes impaired insulin action. Depending upon the severity of the disease the symptoms can be managed through weight loss and diet management, however pharmaceutical intervention is also prevalent. As with other anti-diabetic agents, repaglinide is indicated in clinical situations where a patient's hyperglycaemia can no longer be controlled satisfactorily by diet, weight reduction and exercise alone.

Repaglinide is described as a carbamoylmethyl benzoic acid derivative, typically owing to its structural relationship to meglitinide. Repaglinide's mode of action is exerted by its binding to a receptor site located upon the pancreatic β-cell membrane. Following binding to the β-cell membrane, downstream effects cause ATP-sensitive potassium channels to close. The closing of the potassium channels in turn creates a depolarisation of the β-cell, causing the opening of voltage-sensitive calcium channels. The cooperative closing of potassium channels and opening of calcium channels allows an influx of extracellular calcium ions across the cell membrane. As the intracellular concentration of calcium ions increases this causes the release of previously synthesised insulin, which is stored in secretory vesicles. Consequently, the increased concentration of insulin in the bloodstream leads to increased glucose absorption thus reducing the hyperglycaemic environment.

Repaglinide is named 2-ethoxy-4-[N-[3-methyl-1(S)-[2-(1-piperidinyl)phenyl)butyl]carbamoylmethyl]benzoic acid. Repaglinide is represented structurally by the formulae below.

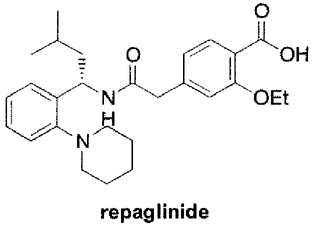

Repaglinide is indicated for the treatment of adult patients with type 2 diabetes mellitus whose hyperglycaemia can no longer be controlled satisfactorily by diet, weight reduction and exercise alone. Repaglinide is also indicated in combination with metformin in adults with type 2 diabetes mellitus whose condition is not satisfactorily controlled with metformin alone. The drug is commercially marketed by Novo Nordisk (under the trade name Prandia®), Actavis and Accord Healthcare. In each case repaglinide is formulated as a tablet in a size amount of 0.5, 1 or 2 mg.

WO 04/101540 details a synthesis of repaglinide:

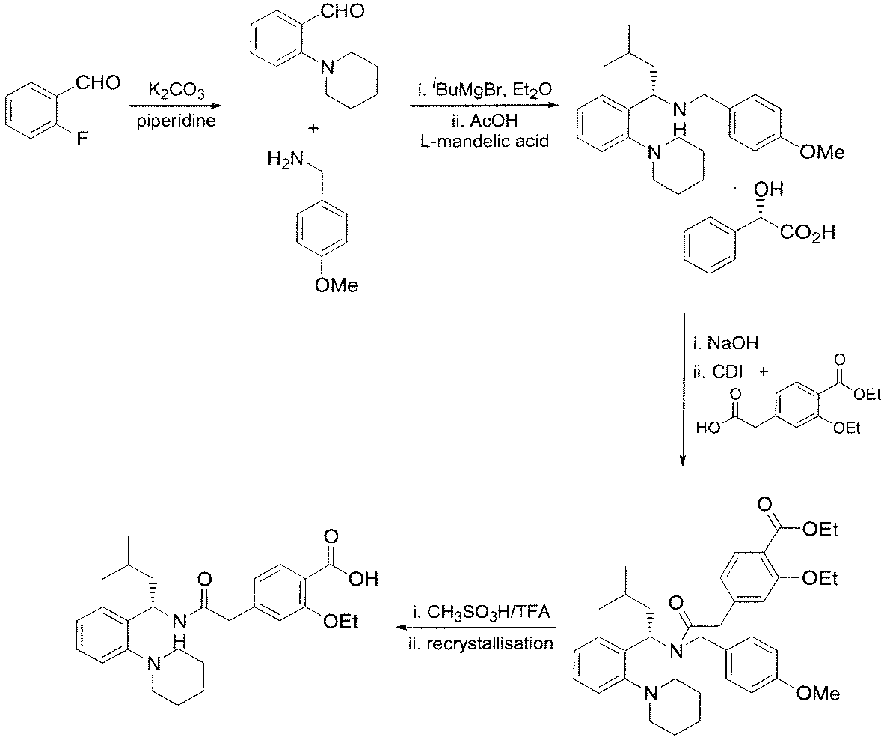

Following a Mannich-type approach, the condensation reaction of the benzaldehyde derivative with p-methoxybenzylamine generates an imine derivative, which then serves as an electrophile for isobutylmagnesium bromide. Following addition of the Grignard, a new racemic (owing to the lack of stereo-selectivity or -specificity of the addition step) stereocentre is generated. The racemic mixture is then resolved via chiral resolution with L-mandelic acid. After obtaining the desired (S) intermediate, trivial manipulations of the scaffold lead to the desired product in three additional steps, and the product is then purified by recrystallisation from an acetonitrile - water mixture. The reaction and purification processes were conducted on a small scale.

WO09/004485 discloses highly pure repaglinide, substantially free from a dimeric impurity, and a process for preparing and isolating thereof. The process for the purification of repaglinide comprises:
a) providing a solution of crude repaglinide in a suitable solvent;
b) admixing the solution of step-(a) with an anti-solvent; and
c) recovering pure repaglinide substantially free of dimer impurity.

Step (a) of providing a solution of crude repaglinide includes dissolving crude repaglinide in the solvent, or obtaining an existing solution from a previous processing step. The solution in step (a) may also be prepared by reacting (S)-3-methyl-1-(2-piperidinophenyl)-1-butylamine with 3-ethoxy-4-ethoxycarbonyl phenyl acetic acid in the presence of a dehydrating agent, optionally in the presence of an organic or inorganic base, in a suitable solvent under suitable conditions, followed by hydrolysis in the presence of an acid or a base to produce a reaction mass containing crude repaglinide followed by usual work up such as washings, extractions etc., and dissolving the resulting crude repaglinide in the solvent at a temperature of above about 25°C. The purification was conducted on a small scale.

The synthetic methodology and purification procedures employed to produce multi-kilogram quantities of a market-approved pharmaceutically active ingredient, for example repaglinide differ greatly from the methodologies employed at the discovery stage. Early stage drug discovery campaigns typically require the production of 5-10 mg of material (per molecule of interest). Such an amount allows for thorough characterisation of physical and chemical parameters, and moreover represents a workable quantity for use in biological assessment. Synthetic chemists operating at the discovery level generally do not assess whether a synthetic route taken to access a molecule of interest, is indeed scalable.

Large-scale chemical synthesis (or process chemistry) concerns the development, optimisation and production of multi-kilogram and multi-tonne quantities of pharmaceutically active ingredients and speciality bulk chemicals. Consequently, and owing to economies of scale, any small improvement in a given reaction or purification parameter is particularly economically significant. Therefore, optimisation of a large-scale synthesis that provides, for example, an increase in chemical yield, decrease in reaction time, decrease in reaction temperature, decrease in amount of catalyst or solvent used, increase in the favourability of reagents, reduction in side-product formation, a more environmentally benign synthesis or increase in chemical purity is of interest both to chemical manufacturers and suppliers. Moreover, step optimisation that reduces the need for multiple or hard-to-perform purifications are particularly beneficial. Conventional column chromatography and high-performance liquid chromatography (HPLC) are ubiquitous techniques, and resemble the cornerstone of the discovery chemist's purification arsenal. Unfortunately, these techniques are unsuitable for the production of multi-kilogram and multi-tonne quantities, yet begrudgingly, such purifications will have to be performed if the target is of high value and no feasible alternative exists. Consequently, any improvement in the ease of purification or isolation, through telescoping (wherein two or more, previously independent synthetic transformations converge into a "single" process and therefore require only one purification step) of synthetic procedures, or the identification of an intermediate for recrystallisation, or precipitation, or removal of impurities through conversion into a transient intermediate, or substitution for a cheaper, more environmentally friendly or less toxic reagent, provide an attractive and economically desirable goal. In situations where more traditional purification procedures yield poor or unsuitable results, it becomes necessary that more innovative solutions are required.

Practical achievement of any such goal is however not straightforward, and even careful optimisation of individual parameters of a synthetic or purification step will often fail to provide a workable advantage within an overall production process.

Although syntheses of repaglinide are established, there remains a need in the art for improving the overall synthesis of repaglinide, improving the purification of repaglinide and increasing the purity of repaglinide.

Accordingly the present invention provides a process for the purification of repaglinide, comprising treating repaglinide with hydrogen peroxide.

The present invention entails a process for the purification of repaglinide, comprising solubilising repaglinide in a solvent, and treating repaglinide with hydrogen peroxide.

Typically, organic drug molecules (i.e. those containing at least carbon and hydrogen) are resistant to solubilisation in aqueous solvents. This is often an inherent property derivable from the hydrophobicity of carbon and hydrogen per se. However drug developers will often seek to avoid overly hydrophilic drug scaffolds, because although offering beneficial solubility profiles, they are often poorly absorbed in the GI tract, where (for absorption) molecules require more lipophilic character. Organic molecules possessing ionisable groups (e.g. a carboxylic acid or amine) offer a solution to the solubility/absorption conundrum because depending on the pH of the environment, they can display high solubility in aqueous matter (e.g. when ionised) and high absorption across lipid membranes (e.g. when neutral/non-ionised).

Repaglinide can be described as a benzoic acid derivative and as such displays some degree of aqueous solubility when ionised. The present purification process occurs in an aqueous solvent, and therefore it is useful to enhance the aqueous solubility of repaglinide, prior to the addition of, and treatment with hydrogen peroxide.

In accordance with the present invention it is preferred where repaglinide is reacted with a base in water, prior to the addition of, and treatment with hydrogen peroxide. Reaction of repaglinide (and repaglinide impurities from previous reaction steps) with a base in water allows the benzoic acid moiety to undergo ionisation (through deprotonation) and thus form a metal carboxylate salt, that is more easily solubilised in water.

Bases suitable for use in accordance with the present invention include, metal carbonates, metal bicarbonates, and metal hydroxides, for example, ammonium carbonate, ammonium hydroxide, barium carbonate, barium hydroxide, calcium carbonate, calcium hydroxide, caesium carbonate, caesium hydroxide, lithium carbonate, lithium hydroxide, magnesium carbonate, magnesium hydroxide, potassium carbonate, potassium hydroxide, sodium carbonate, sodium hydroxide, strontium carbonate and strontium hydroxide. In accordance with the present invention, potassium hydroxide is particularly preferred.

In accordance with the present invention, the process for the purification of repaglinide, comprising treating repaglinide with hydrogen peroxide, is conducted in an aqueous solvent. It is preferred where the process for the purification of repaglinide, comprising treating repaglinide with hydrogen peroxide, is conducted in a solvent and co-solvent.

It is known in the art which particular solvent is likely to be most appropriate and most effective for each category of a reactive chemical transformation. For example, water- or air-sensitive transformations require the use of non-aqueous, non-protic solvents such as acetonitrile, tetrahydrofuran, dioxane, dimethylsulfoxide dichloromethane, chloroform or *N*-methylpyrrolidine. Furthermore, water- or air-sensitive reactions may also require an atmosphere of inert gas (to prevent atmospheric water from compromising a transformation) typical inert gases include nitrogen and argon. Alternatively, reactions requiring the use of an aqueous reagent (e.g. aqueous hydrochloric acid or aqueous sodium hydroxide) may require the use of one or more organic co-solvents. In this context, an organic co-solvent is added to aid the dissolution, or partial dissolution of a less polar organic reagent. Typical organic co-solvents for use with such aqueous reagents include, toluene, alcohols (methanol, ethanol, propanol), tetrahydrofuran and dimethylsulfoxide. Depending upon the particular process, the only limitation in regard to reaction efficacy when using organic co-solvents with aqueous solvents is the requirement for some degree of miscibility of the organic co-solvent with water. To aid problems with miscibility and or reaction efficacy a phase transfer catalyst may be employed (e.g. tetrabutylammonium bromide).

In matters concerning purification as opposed synthetic chemical transformations, it is less apparent which solvents are likely to offer the best results. Typically, the ambiguity surrounding the choice and or mixture of solvent and co-solvent is owing to the variability in the chemical composition of impurities and the physical properties of the target compound.

In accordance with the present invention it is preferred wherein the process for the purification of repaglinide, comprising treating repaglinide with hydrogen peroxide, is conducted in a solvent and co-solvent wherein the process for the purification of repaglinide, comprising treating repaglinide with hydrogen peroxide, is conducted in water and the co-solvent is an alcoholic solvent.

Examples of suitable alcoholic solvents for use as a co-solvent in accordance with the present invention include, *tert*-amyl alcohol, 1,4-butanediol, 1,2,4-butanetriol, *n*-butanol, 2-butanol, *tert*-butyl alcohol, diethylene glycol, ethanol, ethylene glycol, 2-ethylhexanol, glycerol, isobutanol, isopropanol, methanol, 2-(2-methoxyethoxy)ethanol, 2-methyl-1-butanol, 2-methyl-1-pentanol, 3-methyl-2-butanol, neopentyl alcohol, 2-pentanol, 1,3-propanediol, 1-propanol and propylene glycol. Isopropanol is particularly preferred.

It is preferred where the process for the purification of repaglinide, comprising treating repaglinide with hydrogen peroxide, is conducted in water and a co-solvent, wherein the co-solvent is isopropanol.

The process for the purification of repaglinide, comprising treating repaglinide with hydrogen peroxide is advantageously conducted at a temperature obtainable by the application of simple and non-expensive heating technologies. Preferably the process of the present invention is conducted at a temperature from 20°C to 70°C.

The present invention requires non-expensive heating technologies in order to obtain the most preferred reaction temperature of from 55°C to 65°C. Advantageously, the process for the purification of repaglinide requires a relatively short period of heating. Typically the purification process requires of 1 to 5 hours. Most preferably the process requires 3 hours.

The amount of hydrogen peroxide used in accordance with the present invention is typically a molar excess in comparison to the molar amount of repaglinide being purified. That is, if one mole of repaglinide is to be purified, typically one or more molar equivalents of hydrogen peroxide are required.

Hydrogen peroxide is broadly commercially available as a solution in water. Various w/w amounts of hydrogen peroxide in water are marketed, and often the amounts are dependent upon the supplier. Typically, and in accordance with the present invention a 30% w/w solution of hydrogen peroxide in water is preferred.

In accordance with the present invention it is preferred where the process for the purification of repaglinide is conducted wherein 1 to 6 molar equivalents of hydrogen peroxide are used with respect to 1 molar equivalent of repaglinide. It is more preferred where the process for the purification of repaglinide is conducted wherein 2 to 4 molar equivalents of hydrogen peroxide are used with respect to 1 molar equivalent of repaglinide. It is most preferred where the process for the purification of repaglinide is conducted wherein 2.5 to 3.5 molar equivalents of hydrogen peroxide are used with respect to 1 molar equivalent of repaglinide.

In accordance with the present invention the process for the purification of repaglinide, comprising treating repaglinide with hydrogen peroxide, is conducted in water. The process for the purification of repaglinide, comprising treating repaglinide with hydrogen peroxide, is conducted in water and the co-solvent is an alcoholic solvent. It also preferred where the process for the purification of repaglinide, comprising treating repaglinide with hydrogen peroxide, is conducted in water, the co-solvent is an alcoholic solvent and 1 to 6 molar equivalents of hydrogen peroxide are used with respect to 1 molar equivalent of repaglinide. It more preferred where the process for the purification of repaglinide, comprising treating repaglinide with hydrogen peroxide, is conducted in water, the co-solvent is an alcoholic solvent and 2 to 4 molar equivalents of hydrogen peroxide are used with respect to 1 molar equivalent of repaglinide. It is most preferred where the process for the purification of repaglinide, comprising treating repaglinide with hydrogen peroxide, is conducted in water, the co-solvent is an alcoholic solvent and 2.5 to 3.5 molar equivalents of hydrogen peroxide are used with respect to 1 molar equivalent of repaglinide. It is also most preferred where the process for the purification of repaglinide, comprising treating repaglinide with hydrogen peroxide, is conducted in water, the co-solvent is isopropanol and 2.5 to 3.5 molar equivalents of hydrogen peroxide are used with respect to 1 molar equivalent of repaglinide.

The process of the present invention may also include the use of an additive. An additive, with respect to the present process, and chemical reactions per se, should be understood as a species which does not take part in a chemical reaction (i.e. a bond-forming or bond-breaking process). In accordance with the present invention a particularly preferred additive is activated carbon. It is noted that activated carbon is also referred to in the art as both activated charcoal and activated coal.

It is preferred where the process for the purification of repaglinide, comprising treating repaglinide with hydrogen peroxide, is conducted in the presence of an additive.

It is more preferred where the process for the purification of repaglinide, comprising treating repaglinide with hydrogen peroxide, is conducted in the presence of an additive, wherein the additive is activated carbon.

It is also preferred where the process for the purification of repaglinide, comprising treating repaglinide with hydrogen peroxide, is conducted in water, the co-solvent is isopropanol and an additive is present. It is more preferred where the process for the purification of repaglinide, comprising treating repaglinide with hydrogen peroxide, is conducted in water, the co-solvent is isopropanol and an additive is present, wherein the additive is activated carbon.

In accordance with the present invention, repaglinide is provided having a bulk purity of 99.5% or greater as measured by high-performance liquid chromatography.

The term "bulk" should be understood as applying to amounts of repaglinide that weigh 1 kilogram or more, that is amounts that are to be produced at a process chemistry level (i.e. an industrial plant scale process).

In accordance with the present invention, there is also provided a pharmaceutical composition comprising repaglinide having a bulk purity of 99.5% or greater as measured by high-performance liquid chromatography.

The purity of repaglinide can be analysed and measured using high-performance liquid chromatography. Preferred solvents for use in HPLC analysis and purification are solution (A) - aqueous solution comprising distilled H₂O and 4.0 g/dm³ calcium dihydrogen phosphate (adjusted to pH 3.2 with diluted phosphorous acid) and solution (B) - comprising a mixture of solution (A)/MeCN (30:70 v/v). Typically, HPLC analysis can be performed using a Zobrax SB-QA 150 x 4.6mm 5µm column at temp. 45°C, where detection is set at 240nm, and wherein elution is conducted using (solution (A) / solution (B) - gradient (50/50 to 7/93)) over a period of 20 minutes.

In accordance with the present invention, purified repaglinide is obtainable by a process comprising treating repaglinide with hydrogen peroxide. Furthermore, and in accordance with the present invention, purified repaglinide having a purity of 99.5% or greater as measured by high-performance liquid chromatography is obtainable by a process comprising treating repaglinide with hydrogen peroxide.

Following completion of the purification process of the present invention, repaglinide must be retrieved from solution. As previously described, repaglinide can be described as a benzoic acid derivative and as such displays some degree of aqueous solubility when ionised. After solubilising repaglinide as a carboxylate salt in a solvent (e.g. via treatment with a base) and performing the processes of the present invention, repaglinide's carboxylate moiety can be protonated in order to decrease its aqueous solubility and therefore aid precipitation/crystallisation from solution. Typically, after the purification process is completed, the solution is acidified (that is to a pH value below 7.0, and preferably to pH 5.0). Methods for measuring the pH of a solution are well known in the art, and for instance can include a pH meter, litmus paper and universal indicator paper.

In accordance with the present invention, a process for the purification of repaglinide comprising treating repaglinide with hydrogen peroxide can include the following steps:
- solubilising repaglinide in water and heat to 60°C;
- add hydrogen peroxide in water (30% w/w);
- add activated carbon and an alcohol and cool to 25°C;
- acidify to a pH less than pH 7.0; and
- collect purified repaglinide.

The process of the present invention is performed in one pot. The term "one pot" should be understood as referring to a single operational step, i.e. where reagents are added consecutively into one pot, and no intermediate is isolated.

The present invention provides a process for purifying repaglinide, whereby the purification process is conducted in a single vessel (or one-pot). Advantageously, the addition of multiple reagents into a single vessel minimises both the requirement for multiple purification steps and reduces the operational complexity associated with purification procedures conducted using multiple steps in multiple vessels.

This invention provides a process for the purification of repaglinide comprising solubilising repaglinide in water, and treating the repaglinide-containing solution with hydrogen peroxide.

Accordingly, it can be seen that the present invention provides a process for the purification of repaglinide comprising treating repaglinide with hydrogen peroxide.

The present invention will now be described with reference to the examples, which are not intended to be limiting.

### Examples

### Example 1

### (S)-N-[3-methyl-1-[2-(1-piperidinyl)phenyl)butyl]-N-4-methoxybenzylamine

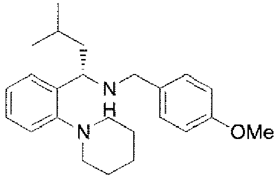

To a suspension of (S)-1-(2-piperidino-phenyl)-3-methyl-1-butyl-N-4-methoxybenzylammonium-L-mandelate (93.2 g) in toluene (540 mL), an aqueous solution of sodium hydroxide (11 g NaOH in 270 mL H₂O) was added. The reaction mixture was stirred for 50 min. After phase separation, a further portion of sodium hydroxide solution (11 g NaOH in 270 mL H₂O) was added. The organic phase was washed with water (90 mL) and concentrated under reduced pressure (p=40mbar; T=55-60°C). The resulting residue was diluted with toluene (120 mL) and again concentrated under reduced pressure (p=40mbar; T=55-60°C). Following concentration under reduced pressure, the dry free amine was obtained. At which point the amine was taken up in toluene (120 mL) in preparation for the next step.

### Example 2

### Ethyl (S)-2-ethoxy-4-{N-[1-(2-piperidinyl)-phenyl-3-methyl-1-butyl]-N-(4-methoxybenzyl) aminocarbonylmethyl}-benzoate

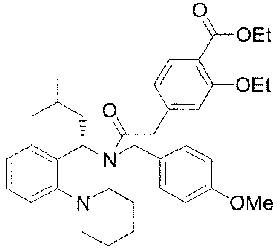

To a suspension of 1,1'-carbonyldiimidazole (40.8 g) in toluene (328 mL) a solution of 3-ethoxy-4-ethoxy-carbonyl-phenyl (46 g) in acetonitrile (200 mL) was added. The reaction mixture was stirred for 30 min. at 20-25°C, and the previously prepared solution of (S)-N-[3-methyl-1-[2-(1-piperidinyl)phenyl)butyl]-N-4-methoxybenzylamine in toluene was added. The reaction was then heated to 45°C, and the temperature was maintained at 45-50°C for 10 hours. The reaction was monitored for conversion of (S)-N-[3-methyl-1-[2-(1-piperidinyl)phenyl)butyl]-N-4-methoxybenzylamine ((S)-amine) (full conversion if (S)-amine ≤ 0.5%). Following full conversion of the (S)-amine, 1,1'-carbonyldiimidazole (0.5 g) was added to reaction mixture and stirred for 2 hours at 45-50°C. After complete condensation, the reaction mixture was cooled to 20-25°C and washed with water (3 x 280 mL). The organic phase was concentrated (p=40mbar; T=55-60°C) and the product was diluted with toluene (120 mL) in preparation for the next step.

### Example 3

### (S)-2-ethoxy-4-{N-[1-(2-piperidinyl)-phenyl-3-methyl-1-butyl]-N-(4-methoxybenzyl) aminocarbonylmethyl}-benzoic acid

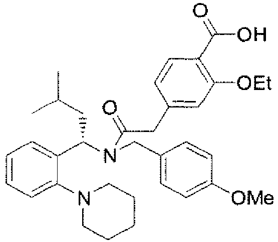

To solution of ethyl (*S*)-2-ethoxy-4-{N-[1-(2-piperidinyl)-phenyl-3-methyl-1-butyl]-N-(4-methoxybenzyl) aminocarbonylmethyl)-benzoate ((*S*)-amide 1) in toluene, water (18 mL) and trifluoroacetic acid (214.6 g) was added. The reaction mixture was heated to 95°C and stirred at 95-100°C for 10 hours (or until full conversation was reached - full conversion if (S)-amide 1 ≤ 2%).

### Example 4

### Crude 2-ethoxy-4-[N-[3-methyl-1(S)-[2-(1-piperidinyl)phenyl]butyl]carbamoyl-methyl]benzoic acid (crude repaglinide)

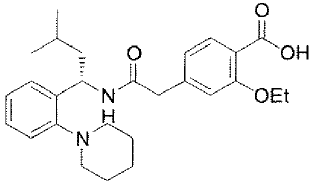

Following de-esterification, the reaction mixture was cooled to 85°C and methanesulfonic acid (66.6 g) was added. The reaction mixture was allowed to stir for 2 hours at 85-90°C, after which it was cooled to 60°C and washed with water (280 mL) at 55-60°C. The reaction mixture was then further diluted with toluene (250 mL) and water (180 mL). Following solvent addition, a 20% aqueous solution of sodium hydroxide (160 mL) was added dropwise thereto (to reach a target pH of 4.9-5.2) at 55-60°C. After phase separation, the organic phase was diluted with toluene (360 mL) and washed with water (4 x 230 mL) at 55-60°C. Water (340 mL) was again added to the organic phase and the reaction was cooled to 42°C. To the biphasic solution (0.05-0.1) g of repaglinide was added to initiate a crystallisation process. The reaction mixture was stirred for a further 2 hours at 40-42°C and then cooled to 15°C (and left for 10 hours). The cream crystals were separated via filtration, and washed with toluene (95 mL) and water (200 mL). The crystalline solids were dried at 50°C to give crude repaglinide. Yield: 67.0 g (82%). Purity: 98.5% by HPLC.

The yield referred to in Example 4 is representative of the total yield over four steps (the synthesis beginning at Example 1).

### Example 5

### Purification of 2-ethoxy-4-[N-[3-methyl-1(S)-[2-(1-piperidinyl)phenyl]butyl]carbamoyl-methyl]benzoic acid (repaglinide)

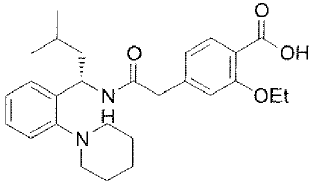

Crude repaglinide (15 g) was added to aqueous solution of potassium hydroxide (6.6 g KOH in 288 mL H₂O). The reaction mixture was heated to 60°C, at which point the repaglinide potassium carboxylate salt was dissolved in water. After 1 hour of stirring at 60°C hydrogen peroxide (30% H₂O₂, 10 mL) was added dropwise to the mixture (within 1 hour) and stirred for 3 hours at 57-60°C. Next, an aqueous solution of hydrochloric acid (1:1 H₂O:HCl 10 mL) was added dropwise (to adjust the pH to 9-10). Isopropanol (150 mL) and activated carbon (1.05 g) were then added and the reaction mixture was cooled to 25°C (over 1 hour). The activated carbon was filtered off and washed with an isopropanol-water solution 1:1 (15 mL). After filtration, isopropanol (96 mL) was added and reaction mixture was heated to 30°C. A solution of hydrochloric acid (1:1 H₂O:HCl 8-9 mL) was added dropwise (to adjust the pH to 4.9-5.2) within 30 minutes, and the resulting suspension of repaglinide in isopropanol:water was cooled to 15°C (over 10 hours). Repaglinide was filtered off and washed with solution of isopropanol:water (20 mL isopropanol: 30 mL water) and water (60 mL). The washed solids were dried at 50°C to give pure repaglinide as colourless crystals. Yield: 12.98 g (86.5%). Purity: 99.6% by HPLC, Mp 132-133°C.

**Table 1**

| Raw material requirement for the purification of 1kg of crude repaglinide | | | |
|---|---|---|---|
| **No.** | **Reagent** | **Amount (g, mol) (used in Example 5)** | **Amount (kg) required to purify 1kg of repaglinide** |
| 1. | Repaglinide crude | 15.0, 0.03 | 1.16 |
| 2. | Potassium hydroxide | 6.6, 0.12 | 0.51 |
| 3. | Hydrogen peroxide (30%) | 11.0, 0.10 | 0.85 |
| 4. | Water | 395.5, 21.90 | 30.48 |
| 5. | Isopropanol | 214.7, 3.57 | 18.61 |
| 6. | Carbon activated | 0.75, 0.06 | 0.06 |
| 7. | Hydrochloric acid 35% | 12.0, 0.11 | 0.92 |
| 8. | Purity | **99.6%** (HPLC), max. impurity: 0.10% (HPLC) | |
| 9. | Yield | 12.98 g **(86.5%)** | |
| 10. | Form | Colourless crystals | |
| 11. | Polymorphic form | M.p. 132-133°C | |

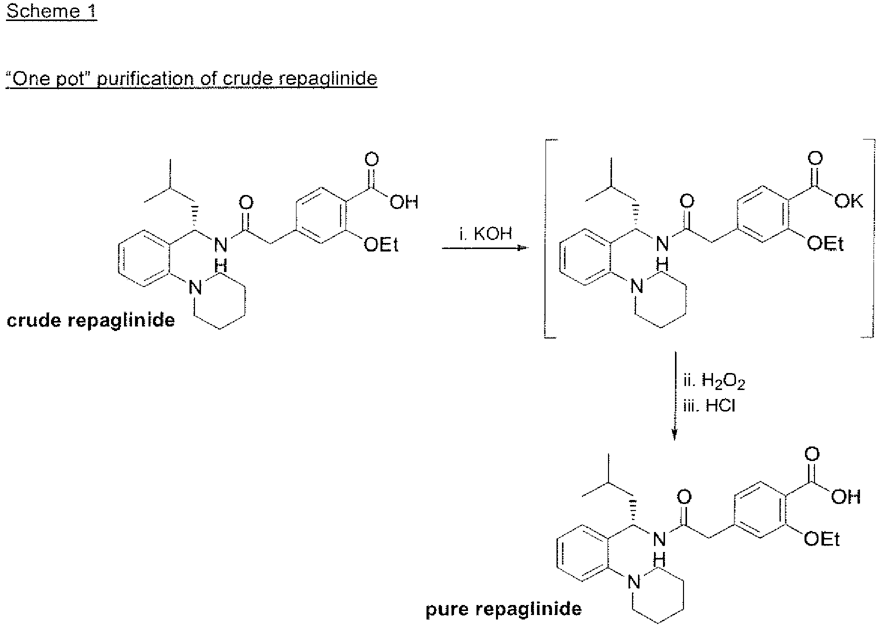

## Claims

1. A process for the purification of repaglinide comprising solubilising repaglinide in an aqueous solvent, and treating repaglinide with hydrogen peroxide.

2. A process as claimed in claim 1 comprising an aqueous solvent and an alcoholic co-solvent.

3. A process as claimed in claim 2 wherein the aqueous solvent is water, and the alcoholic co-solvent is isopropanol.

4. A process as claimed in any one of the preceding claims, wherein repaglinide is treated with hydrogen peroxide at a temperature of from 20 to 70°C.

5. A process as claimed in any one of the preceding claims, wherein treatment occurs over a period of 1 to 5 hours.

6. A process as claimed in any one of the preceding claims, wherein 1 to 6 molar equivalents of hydrogen peroxide are used with respect to 1 molar equivalent of repaglinide.

7. A process as claimed in claim 6, wherein 2 to 4 molar equivalents of hydrogen peroxide are used with respect to 1 molar equivalent of repaglinide.

8. A process as claimed in any one of the preceding claims, wherein 2.5 to 3.5 molar equivalents of hydrogen peroxide are used with respect to 1 molar equivalent of repaglinide.

9. A process as claimed in any one of the preceding claims further comprising activated carbon.

10. A process as claimed in any one of the preceding claims, wherein repaglinide is provided with a bulk purity of 99.5% or greater as measured by high-performance liquid chromatography.

## Patentansprüche

1. Verfahren zur Reinigung von Repaglinid, das die Solubilisierung von Repaglinid in einem wässrigen Lösungsmittel und die Behandlung von Repaglinid mit Wasserstoffperoxid umfasst.

2. Verfahren gemäß Anspruch 1, das ein wässriges Lösungsmittel und ein alkoholisches Co-Lösungsmittel umfasst.

3. Verfahren gemäß Anspruch 2, wobei das wässrige Lösungsmittel Wasser und das alkoholische Co-Lösungsmittel Isopropanol ist.

4. Verfahren gemäß einem der vorstehenden Ansprüche, wobei Repaglinid mit Wasserstoffperoxid bei einer Temperatur von 20 bis 70°C behandelt wird.

5. Verfahren gemäß einem der vorstehenden Ansprüche, wobei die Behandlung während eines Zeitraums von 1 bis 5 Stunden erfolgt.

6. Verfahren gemäß einem der vorstehenden Ansprüche, wobei 1 bis 6 Moläquivalente von Wasserstoffperoxid bezogen auf 1 Moläquivalent Repaglinid verwendet werden.

7. Verfahren gemäß Anspruch 6, wobei 2 bis 4 Moläquivalente von Wasserstoffperoxid bezogen auf 1 Moläquivalent Repaglinid verwendet werden.

8. Verfahren gemäß einem der vorstehenden Ansprüche, wobei 2,5 bis 3,5 Moläquivalente von Wasserstoffperoxid bezogen auf 1 Moläquivalent Repaglinid verwendet werden.

9. Verfahren gemäß einem der vorstehenden Ansprüche, das ferner Aktivkohle umfasst.

10. Verfahren gemäß einem der vorstehenden Ansprüche, wobei Repaglinid mit einem Reinheitsgrad der Bulkware von 99,5% oder mehr, gemessen mit Hilfe von Hochleistungsflüssigkeitschromatographie, bereitgestellt wird.

## Revendications

1. Procédé pour la purification du répaglinide comprenant la solubilisation du répaglinide dans un solvant aqueux et le traitement du répaglinide avec du peroxyde d'hydrogène.

2. Procédé selon la revendication 1 comprenant un solvant aqueux et un cosolvant alcoolique.

3. Procédé selon la revendication 2 dans lequel le solvant aqueux est de l'eau et le cosolvant alcoolique de l'isopropanol.

4. Procédé selon l'une quelconque des revendications précédentes dans lequel le répaglinide est traité avec du peroxyde d'hydrogène à une température comprise entre 20 et 70°C.

5. Procédé selon l'une quelconque des revendications précédentes dans lequel le traitement a lieu sur une période allant de 1 à 5 heures.

6. Procédé selon l'une quelconque des revendications précédentes dans lequel 1 à 6 équivalents molaires de peroxyde d'hydrogène sont utilisés pour 1 équivalent molaire de répaglinide.

7. Procédé selon la revendication 6 dans lequel 2 à 4 équivalents molaires de peroxyde d'hydrogène sont utilisés pour 1 équivalent molaire de répaglinide.

8. Procédé selon l'une quelconque des revendications précédentes dans lequel 2,5 à 3,5 équivalents molaires de peroxyde d'hydrogène sont utilisés pour 1 équivalent molaire de répaglinide.

9. Procédé selon l'une quelconque des revendications précédentes comprenant par ailleurs du charbon actif.

10. Procédé selon l'une quelconque des revendications précédentes dans lequel le répaglinide est fourni à une pureté en vrac supérieure ou égale à 99,5% telle que mesurée par la chromatographie en phase liquide à haute performance.
